# FASCICULE DE BREVET EUROPEEN

(11) **EP 1 789 573 B1**
(45) Date de publication et mention de la délivrance du brevet: **18.03.2009**
(21) Numéro de dépôt: 05800539.8
(22) Date de dépôt: 14.09.2005
(51) Int. Cl.: C12Q 1/14, C12Q 1/34

(54) **PROCEDE DE DETECTION DE STREPTOCOCCUS AGALACTIAE EN UTILISANT L'ACTIVITE ALPHA-GLUCOSIDASE**
VERFAHREN FÜR DEN NACHWEIS VON STREPTOCOCCUS AGALACTIAE MITTELS ALPHA-GLUCOSIDASE-AKTIVITÄT
METHOD FOR DETECTING STREPTOCOCCUS AGALACTIAE USING ALPHA-GLUCOSIDASE ACTIVITY

(30) Priorité: 16.09.2004 FR 0452066
(43) Date de publication de la demande: 30.05.2007
(73) Titulaire: BIOMERIEUX, 69280 Marcy-L'Etoile (FR)
(72) Inventeur: ROBICHON, Denis, F-01150 BLYES (FR)
(74) Mandataire: Sprugnoli, Claude Louis Victor
(86) Numéro de dépôt international: PCT/FR2005/050740
(87) Numéro de publication internationale: WO 2006/032810

(56) Documents cités:
- WO-A-01/30794
- US-A- 5 888 760
- US-A1- 2002 147 317

## Description

La présente invention concerne le domaine de la détection et l'identification de *Streptococcus agalactiae*. Plus particulièrement, l'invention concerne l'utilisation de substrats enzymatiques d'α-glucosidase pour la détection et l'identification de *Streptococcus agalactiae.*

Le genre *Streptococcus* comporte de nombreuses espèces très répandues dans la nature, sur la peau et les muqueuses de l'homme et des animaux et sont à l'origine de multiples infections. Ce sont des bactéries ubiquistes que l'on retrouve à l'état libre dans le milieu extérieur (sol, air, eau), à l'état saprophyte ou à l'état de commensal chez l'homme et les animaux. Leurs localisations sont le rhinopharynx pour les streptocoques des groupes A, C, G, H et *salivarius*, l'intestin pour les streptocoques fécaux du groupe D et la cavité vaginale pour les streptocoques du groupe B. Leur rôle pathogène est extrêmement varié et dépend des espèces en cause et de leur localisation dans l'organisme.

Les streptocoques sont des coques Gram+, de 0,5 à 1 µm de diamètre, présentant un groupement en chaînette et immobiles. Catalase négative, à métabolisme fermentant, ils sont anaérobies facultatifs et sont sensibles aux variations de température (croissance optimale 37°C) et aux variations de pH (pH optimal 7).

*Streptococcus agalactiae* (ou streptocoque B) est reconnu comme l'un des principaux agents infectieux responsables de la mammite chez les bovidés. Chez l'homme, c'est essentiellement un saprophyte des voies génitales de la femme (vagin), mais il se retrouve également dans le rhinopharynx et dans l'intestin, notamment le rectum. Chez les adultes, la colonisation demeure souvent asymptomatique, mais *Streptococcus agalactiae* peut être responsable de septicémies, de pneumonies, de méningites, d'arthrites, d'infections urinaires et de suppurations profondes. Chez la femme enceinte ou après l'accouchement, l'infection peut conduire à des endométrites et à une stérilité.

Chez le nouveau-né, la contamination se produit *in utero* ou, le plus souvent, lors de l'accouchement par inhalation du liquide amniotique ou de secrétions vaginales. Une infection précoce apparaît souvent dès la naissance ou dans les premières heures de la vie. L'infection précoce est favorisée par la pre-maturité, la rupture des membranes et une forte colonisation du vagin de la mère. Le taux de mortalité dans ce type d'infection est très élevé (> 50%). Les infections tardives se traduisent généralement par des méningites (méningite du nourrisson) et des arthrites.

Le dépistage systématique du portage de *Streptococcus agalactiae* est recommandé en fin de grossesse, idéalement entre 34 et 38 semaines d'aménorrhée (35-37 semaines de grossesse), en raison notamment de sa prévalence (10% en France, soit au moins 75 000 femmes enceintes/an) et de ses conséquences lors des accouchements à terme, ce qui en fait un problème de santé publique.

Des milieux sélectifs et/ou des milieux permettant une orientation du diagnostic sont disponibles dans le commerce. Toutefois, ces milieux ont pour inconvénient qu'ils ne se suffisent pas à eux seuls pour le diagnostic de *Streptococcus agalactiae* et qu'il est nécessaire d'effectuer des tests complémentaires tels que la mise en évidence de l'antigène du groupe B de Lancefield (polysaccharide avec présence dominante de rhamnose) et l'hydrolyse de l'hippurate (bouillon à l'hippurate).

Les milieux sélectifs les plus couramment utilisés sont le bouillon de Todd-Hewitt, bouillon d'enrichissement destiné à la recherche des streptocoques du groupe B chez la femme enceinte. Ce bouillon contient différents antibiotiques inhibant la plupart des germes Gram négatif de la flore d'accompagnement, tels que l'acide nalixidique et la gentamycine, ou l'acide nalixidique, la polymyxine et le cristal violet.

Après l'étape d'enrichissement, le bouillon de Todd-Hewitt complémenté en antibiotiques doit être repiqué sur des milieux destinés à la recherche des streptocoques (voir recommandations du CDC (Center for Disease Control), MMWR (Morbidity and Mortality Weekly Report), 16 août 2002, Vol. 51, n° RR-11).

Le milieu de Lim est un variant du bouillon Todd-Hewitt et il contient 1% d'extrait de levure, de l'acide nalixidique et de la colistine.

Une gélose Columbia contenant 5% de sang est également utilisée et permet notamment la mise en évidence du caractère β-hémolytique de *Streptococcus agalactiae.* Toutefois, ce caractère n'apparaît pas toujours : le halo d'hémolyse autour des colonies peut être étroit, donnant plutôt l'aspect α-, voire γ-hémolyitque. En revanche, ce caractère devient net si au voisinage des colonies de *Streptococcus agalactiae*, se trouvent des colonies de *Staphylococcus aureus* (Camp-Factor).

Ces milieux sélectifs ont pour inconvénients qu'ils doivent être complétés par des tests biochimiques et/ou immunologiques.

Actuellement, le seul milieu sélectif prêt à l'emploi disponible dans le commerce, permettant l'isolement et l'identification directe de *Streptococcus agalactiae* à partir de prélèvements recto- vaginaux est le milieu Granada (Biolys SA). Ce milieu a pour caractéristique qu'il favorise la production d'un pigment caroténoïde par les souches de *Streptococcus agalactiae* du fait de la présence dans le milieu d'amidon soluble, protéose peptone n°3, glucose, pyruvate de sodium, sulfate de magnésium, méthotrexate, colistine, cristal violet, agar, sérum de cheval, Na₂HPO₄ anhydre, métronidazole, MOPS (acide morpholinopropanesulfonique) hémisodique, eau distillée et incubation en anaréobiose. Ce milieu a donc pour inconvénient que la détection directe de *Streptococcus agalactiae* se fait en condition anaérobie, ce qui n'est pas aisé à mettre en oeuvre. Par ailleurs, aucun milieu de détection contenant un ou plusieurs substrats enzymatiques n'est disponible.

WO0130794 montre seulement en termes générales qu'on pourrait détecter les microbes comme Escherichia coli, Shigella, Salmonella, Proteeae, Pseudomonas aeruginosa, Neisseria meningitidis, Enterococcus, Staphylococcus aureus, Bacillus cereus, Listera monocytogenes, Streptococcus pyogenes, Streptococcus agalactiae, Candida albicans, Candida glabrata, Candida tropicalis, Cryptococcus neoformans et Aspergillus fumigatus par détecter des enzymes comme la β-glucuronidase, la β-galactosidase, la 6-phosphogalactohydrolase, l'a-galactosidase, l'α-amylase, l'α-glucosidase, la β-glucosidase, les hexosaminidases comme la N-acetyl-β-glucosaminidase ou la N-acetyl-β-galactosaminidase, de la famille des estérases, des lipases, des phosphatases, des sulfatases, des DNases, des peptidases et des proteases.

La Demanderesse a mis en évidence contre toute attente qu'il était possible d'utiliser des substrats enzymatiques, en particulier des substrats enzymatiques d'α-glucosidase, pour la détection spécifique et l'identification de *Streptococcus agalactiae.*

En effet, de façon surprenante, non seulement les substrats enzymatiques sont utilisés par les *Streptococcus agalactiae* permettant leur révélation, par exemple en produisant une modification de la coloration des colonies dans le milieu lorsqu'on utilise un substrat enzymatique chromogène, sans que la coloration ne diffuse dans le milieu réactionnel, donc concentrée au niveau des colonies, mais ces molécules n'ont aucun effet néfaste sur la croissance de ces bactéries.

Ainsi la présente invention a pour objet un procédé de détection spécifique et d'identification de *Streptococcus agalactiae*, **caractérisé en ce qu**'il utilise un milieu réactionnel comprenant au moins un substrat enzymatique d'α-glucosidase.

Les substrats enzymatiques d'α-glucosidase appropriés aux fins de l'invention sont tout substrat connu de l'homme du métier permettant de mettre en évidence une telle activité enzymatique. De tels substrats peuvent être par exemple chromogéniques ou fluorescents et sont décrits par exemple dans l'article de Rice P. et al. (2000). A rapid biochemical test to aid identification of Mycoplasma mycoides subsp. mycoides small colony (SC) strains. Lett. Appl. Microbiol.1:70-74 ou dans le catalogue BIOSYNTH, Substrates and Reagents ou www.biosynth.com ou dans le catalogue GLYCOSYNTH, enzyme substrates catalogue ou www.glycosynth.co.uk.

A titre d'exemple, on peut citer les substrats à base de dérivés d'indoxyle, les substrats à base de dérivés d'umbelliférone et les substrats à base de dérivés de naphtol.

Selon un mode de réalisation préféré de l'invention, le substrat enzymatique approprié aux fins de l'invention est un substrat à base de dérivés d'indoxyle.

Des exemples de tels dérivés d'indoxyle comprennent les dérivés de 3-indolyl-α-D-glucopyranoside, de préférence des dérivés halogénés de ces composés.

A titre d'exemples de dérivés de 3-indolyl-α-D-glucopyranoside halogénés, on peut citer le 6-bromo-3-indolyl-α-D-glucopyranoside, le 6-chloro-3-indolyl-α-D-glucopyranoside, le 5-bromo-6-chloro-3-indolyl-α-D-glucopyranoside, le 5-bromo-4-chloro-3-indolyl-α-D-glucopyranoside et le 5-bromo-4-chloro-3-indolyl-N-méthyl-α-D-glucopyranoside, ce dernier composé étant particulièrement préféré.

Le milieu réactionnel tel qu'utilisé dans le procédé de l'invention est donc un milieu réactionnel de détection du fait de la présence du substrat enzymatique.

Ce milieu réactionnel peut soit servir uniquement de milieu de révélation, soit de milieu de culture et de révélation. Dans le premier cas, la culture des microorganismes est effectuée avant ensemencement et, dans le deuxième cas, le milieu réactionnel constitue également le milieu de culture.

Le milieu réactionnel peut être solide, semi-solide ou liquide. Par milieu solide ou semi- solide, on entend par exemple un milieu gélifié.

L'agar est le milieu traditionnel solide en microbiologie pour la culture des microorganismes, mais il est possible d'utiliser de la gélatine ou de l'agarose. Un certain nombre de préparation sont disponibles dans le commerce, comme par exemple l'agar Columbia, la gélose Trypcase-soja, la gélose Mac Conkey, la gélose Sabouraud ou plus généralement celles décrites dans le Handbook of Microbiological Media (CRC Press).

La quantité d'agar dans le milieu réactionnel est de 2 à 40g/l. Pour les milieux solides, la quantité d'agar est de préférence de 9 à 25g/l, de préférence encore de 12 à 14g/l. Pour les milieux semi-solides, la quantité d'agar est de préférence de 2 à 6g/l.

Les substrats enzymatiques de l'invention sont utilisables dans une large gamme de pH, notamment entre pH 5,5 et 10.

La concentration du substrat enzymatique dans le milieu réactionnel est comprise entre 10 et 2000 mg/l, de préférence entre 50 et 500 mg/l, de préférence encore entre 100 et 400 mg/l, ce qui constitue un mode de réalisation préféré de l'invention.

Bien entendu, l'homme du métier déterminera la concentration de substrat enzymatique dans le milieu dans cette gamme en fonction du substrat choisi. Ainsi, dans la mesure où le substrat enzymatique utilisé est le 5-bromo-4-chloro-3-indolyl-N-méthyl-α-D-glucopyranoside, on préfère une concentration comprise entre 130 et 300 mg/l.

Le milieu réactionnel utile aux fins de l'invention peut également comprendre d'autres composants utiles pour améliorer la spécificité et la sensibilité du procédé de l'invention.

Le milieu réactionnel peut également contenir un mélange d'inhibiteurs pour inhiber ou limiter la croissance des souches indésirables, telles que les souches Faux positif, par exemple *Candida* ou *Staphylococcus saprophyticus*, sans modifier la sensibilité de détection du milieu.

A ce titre, le mélange réactionnel peut contenir un mélange d'antibiotiques. L'ajout d'antibiotiques dans le milieu réactionnel permet entre autre un gain de temps car l'identification de *Streptococcus agalactiae* se fait directement.

Des exemples d'antibiotiques qui conviennent aux fins de l'invention comprennent l'aztréonam et l'amphotéricine B. Ces antibiotiques sont disponibles dans le commerce auprès de ICN, Squibb ou Sigma.

Ainsi, selon un mode de réalisation de l'invention, le milieu réactionnel utilisé dans le procédé de détection spécifique et d'identification de *Streptococcus agalactiae* comprend également un mélange d'aztréonam et d'amphotéricine B.

La quantité de chaque antibiotique dans le milieu réactionnel varie en fonction de l'antibiotique concerné et sera facilement déterminée par l'homme du métier.

Ainsi, par exemple, la concentration en aztréonam peut être comprise entre 0,01 et 0,08 g/l et la concentration en amphotéricine B peut être comprise entre 0,002 et 0,006 g/l.

Un mélange préféré de ces antibiotiques contient 0,064 g/l d'aztréonam et 0,004 g/l d'amphotéricine B.

Le milieu réactionnel utilisé dans le procédé de l'invention peut également comprendre au moins un autre substrat spécifique d'une activité enzymatique différente de celle détectée par le substrat d'α-glucosidase. L'hydrolyse enzymatique du ou des autres substrats génère un signal détectable, différent du signal détecté par le substrat d'α-glucosidase, comme par exemple des produits colorés ou fluorescents différents, pour permettre la mise en évidence comme la détection et/ou l'identification et/ou la quantification de *Streptococcus agalactiae* en améliorant la spécificité de mise en évidence.

A titre d'autre substrat spécifique, on peut citer les substrats de β-cellobiosidase, de β-glucosidase, de β-glucosaminidase et tout autre substrat enzymatique connu de l'homme du métier permettant d'éliminer les faux positifs.

Selon un mode de réalisation préféré, le milieu réactionnel comprend également au moins un autre substrat d'activité enzymatique différente, de préférence choisi parmi les substrats de β-cellobiosidase et les substrats de β-glucosaminidase.

L'utilisation d'un substrat de β-cellobiosidase tel que le 6-chloro-3-indolyl-β-D-cellobioside permet d'éliminer les espèces faux positif, telles que *Enteroccocus faecalis*, *Listeria monocytogenes* et *Enterobacter claocae*, sans dégrader la sensibilité de détection de l'activité α-glucosidase des *Streptococcus agalactiae.*

L'utilisation d'un substrat de glucosaminidase tel que le 6-chloro-3-indclyl-β-N-acétyl-glucosaminide ou le 5-bromo-6-chloro-3-indolyl-β-N-acétyl-glucosaminide permet d'éliminer la détection de *Enteroccocus faecalis, Enteroccocus faecium* et *Enterobacter claocae*.

La concentration de l'autre substrat enzymatique spécifique est généralement comprise entre 0,01 et 2 g/l. L'homme du métier pourra déterminer facilement une telle concentration en fonction du substrat utilisé.

Lorsqu'on utilise le 6-chloro-3-indolyl-β-D-cellobioside ou le 6-chloro-3-indolyl-β-N-acétyl-glucosaminide, leur concentration dans le milieu est de préférence de 0,4 g/l.

Le milieu réactionnel peut également comprendre un ou plusieurs éléments en combinaison, tels que des acides aminés, des peptones, des hydrates de carbone, des nucléotides, des minéraux, des vitamines, des tensioactifs, des tampons, des sels de phosphate, d'ammonium, de sodium, de métaux. Des exemples de milieux sont décrits dans les demandes de brevet de la Demanderesse, EP 656 421 et WO99/09 207.

La mise en oeuvre du procédé de l'invention peut être effectuée selon les étapes suivantes consistant à :
a) inoculer un milieu réactionnel tel que défini précédemment, avec tout ou partie de l'échantillon,
b) incuber le milieu inoculé,
c) révéler la présence d'au moins une activité α-glucosidase seule ou en combinaison avec au moins une autre activité enzymatique différente d'une activité α-glucosidase,
ce qui constitue un autre objet de l'invention.

Les étapes d'inoculation et d'incubation sont largement connues de l'homme du métier.

Par exemple, la température d'incubation peut être de 37°C. S'agissant de l'atmosphère d'incubation, elle est préférentiellement aérobie.

La révélation est mise en oeuvre à l'oeil nu par visualisation d'un changement de coloration ne diffusant pas dans le milieu réactionnel, donc concentrée au niveau des colonies. Dans le cas de la révélation de la fluorescence, on utilise les dispositifs de lecture de la fluorescence connus de l'homme du métiers.

Les échantillons biologiques à analyser sont tout échantillon clinique susceptible de contenir des *Streptococcus agalactiae*, comme un prélèvement vaginal, un prélèvement d'urine ou tout autre échantillon dont l'analyse peut aider un clinicien à poser un diagnostic.

L'invention sera mieux comprise à l'aide des exemples suivants donnés à titre illustratif et non limitatif.

### Exemple 1: Détection de Streptococcus agalactiae à l'aide de substrats enzymatiques s d'α-glucosidase

### 1.1 Préparation des milieux réactionnels

On a préparé les milieux réactionnels en mélangeant de l'extrait coeur-cervelle (4,84 g/l ; Solabia), de l'infusion de viande (1,96 g/l ; Solabia), de la biothione (1 g/l; Solabia), de la biotrypcase (7,2 g/l ; Solabia), du carbonate de sodium (0,3 g/l ; VWR), du pyruvate de sodium (2 g/l ; Fluka), du tampon HEPES (0,4 g/l; Sigma), de la peptone de lactalbumine (2 g/l; DMV), du glucose (1 g/l ; Merck), de l'agar américain (2 g/l ; Sobigel) et de l'agar européen (12 g/l ; Roko).

Après autoclavage 15 min à 121°C, on a ajouté un substrat enzymatique d'α-glucosidase tel qu'indiqué ci-dessous à raison de 0,1 g/l ; puis on a refroidi au bain-marie à 50°C.
- 6-bromo-3-indolyl-α-D-glucopyranoside (RedA-α-Glu ; Inalco),
- 6-chloro-3-indolyl-α-D-glucopyranoside (Rose-α-Glu ; Inalco),
- 5-bromo-6-chloro-3-indolyl-α-D-glucopyranoside(Magenta-α-Glu ; Glycosynth),
- 5-bromo-4-chloro-3-indolyl-a-D-glucopyranoside (X-α-Glu ; Biosynth) et
- 5-bromo-4-chloro-3-indolyl-N-méthyl-α-D-glucopyranoside (GreenA-α-Glu ; Inalco).

On a ensuite coulé les milieux en boite de Petri pour l'inoculation ultérieure avec des souches de bactéries.

### 1.2 Ensemencement des souches de microoganismes

Huit souches de *Streptococcus agalactiae* issues de la collection de la Demanderesse, mises en suspension dans de l'eau physiologique, ont été ensemencées pour donner des colonies isolées sur chacun des milieux. Les boîtes ont été incubées à 37 °C pendant 48 heures. Les colonies formées ont été examinées visuellement après 18, 24 et plus de 40 heures d'incubation. La coloration de ces colonies, la croissance, ainsi que l'intensité de cette coloration ont été notées.

### 1.3 Résultats

Les résultats sont donnés dans le tableau 1 ci-après et sont exprimés :
- en croissance (C) avec indication de la taille en mm,
- en intensité (I) de coloration en se basant sur une échelle arbitraire allant de 0 à 4, 0 correspondant à une absence d'activité et 4 correspondant à la présence d'une coloration très intense,
- en couleur (Co) avec T= Turquoise, R= Rose ou Rouge, V=vert, Mg=magenta,
- selon le temps d'incubation en heures (T).

Les résultats tels qu'indiqués dans le tableau 1 mettent en évidence que les streptocoques B peuvent être détectés en utilisant un substrat enzymatique d'α-glucosidase, au moins à partir de 18h d'incubation.

| | | **Milieu 1** | | | **Milieu 2** | | | **Milieu 3** | | | **Milieu 4** | | | **Milieu 5** | | |
|---|---|---|---|---|---|---|---|---|---|---|---|---|---|---|---|---|
| | | **X-alpha-Glu** | | | **RedA-alpha-Glu** | | | **GreenA-alpha-Glu** | | | **rose-alpha-Glu** | | | **Magenta-alpha-Glu** | | |
| **Souches (n°d'accès)** | **T** | **C** | **1** | **Co** | **C** | **I** | **Co** | **C** | **I** | **Co** | **C** | **I** | **Co** | **C** | **I** | **Co** |
| *Streptococcus agalactiae* (7611003) | 18 | 1 | 2,3 | T | 1 | 0,5 | R | 1 | 2 | V | 1 | 0,3 | R | 0,5 | 1 | Mg |
| | 24 | 1,2 | 2,7 | T | 1 | 0,8 | R | 1,3 | 2 | V | 1,3 | 0,6 | R | 0,7 | 2 | Mg |
| | >40 | 1,5 | 3 | T | 1,3 | 2 | R | 1,7 | 3 | V | 1,7 | 1,3 | R | 0,7 | 3 | Mg |
| *Streptococcus agalactiae* (7701031) | 18 | 0,5 | | | 0,5 | | | 0,5 | | | 0,5 | | | 0,5 | | |
| | 24 | 0,7 | | | 0,7 | | | 0,7 | 0,3 | V | 0,7 | | | 0,5 | | |
| | >40 | 1,2 | | | 1,2 | 0,5 | R | 1,5 | 0,8 | V | 1,2 | 0,5 | R | 0,8 | 2 | Mg |
| *Streptococcus agalactiae* (7702055) | 18 | 0,7 | 0,5 | T | 0,7 | | | 0,7 | 1 | V | 0,7 | 0,1 | R | 0,5 | 0,5 | Mg |
| | 24 | 1 | 1 | T | 1 | 0,3 | R | 0,8 | 2 | V | 1 | 0,1 | R | 0,8 | 0,6 | Mg |
| | >40 | 1,7 | 3 | T | 1,7 | 2,3 | R | 1,7 | 3 | V | 1,7 | 1 | R | 1 | 3 | Mg |
| *Streptococcus agalactiae* (8709013) | 18 | 0,4 | 0,1 | T | 0,5 | | | 0,4 | 0,5 | V | 0,4 | | | 0,4 | 0,5 | Mg |
| | 24 | 0,5 | 0,6 | T | 0,5 | | | 0,5 | 1 | V | 0,5 | | | 0,5 | 1,3 | Mg |
| | >40 | 0,7 | 2 | T | 0,7 | 0,8 | R | 0,7 | 2,7 | V | 0,7 | 0,5 | R | 0,7 | 2,7 | Mg |
| *Streptococcus agalactiae* (8904053) | 18 | 0,7 | 0,3 | T | 0,7 | | | 0,7 | 0,5 | V | 0,7 | 0,1 | R | 0,7 | 0,6 | Mg |
| | 24 | 0,7 | 0,6 | T | 0,7 | 0,3 | R | 0,7 | 0,8 | V | 0,7 | 0,3 | R | 0,7 | 0,6 | Mg |
| | >40 | 0,8 | 2,3 | T | 0,8 | 1,7 | R | 1 | 2,3 | V | 0,8 | 1 | R | 0,7 | 3 | Mg |
| *Streptococcus agalactiae* (0008200) | 18 | 0,7 | 0,3 | T | 0,7 | 0,1 | R | 0,7 | 0,5 | V | 0,5 | 0,1 | R | 0,5 | 0,5 | Mg |
| | 24 | 0,7 | 0,6 | T | 0,7 | 0,3 | R | 0,7 | 0,8 | V | 0,5 | 0,3 | R | 0,5 | 0,8 | Mg |
| | >40 | 0,8 | 2,3 | T | 0,7 | 1,7 | R | 0,7 | 2 | V | 0,5 | 0,5 | R | 0,5 | 3 | Mg |
| *Streptococcus agalactiae* (0008206) | 18 | 0,7 | 0,3 | T | 0,7 | 0,3 | R | 0,7 | 0,6 | V | 0,7 | 0,1 | R | 0,7 | 0,6 | Mg |
| | 24 | 0,7 | 1 | T | 0,8 | 0,5 | R | 0,7 | 1 | V | 0,8 | 0,3 | R | 0,7 | 0,8 | Mg |
| | >40 | 0,8 | 2 | T | 1,2 | 2 | R | 0,8 | 2 | V | 1,2 | 0,8 | R | 0,7 | 3 | Mg |
| *Streptococcus agalactiae* (0101060) | 18 | 0,4 | 2,7 | T | 0,4 | 0,5 | R | 0,3 | 0,8 | V | 0,5 | 0,3 | R | 0,4 | 2,7 | Mg |
| | 24 | 0,5 | 3 | T | 0,5 | 0,8 | R | 0,4 | 1,7 | V | 0,7 | 0,6 | R | 0,4 | 2,7 | Mg |
| | >40 | 0,7 | 4 | T | 0,7 | 3 | R | 0,5 | 3,5 | V | 0,8 | 2,3 | R | 0,5 | 4 | Mg |

### Exemple 2 : Modification de la concentration de substrat enzymatique d'α-glucosidase

On a répété le mode opératoire ci-dessus, à ceci près qu'on a fait varier la concentration en substrat enzymatique.

Le tableau 2 ci-dessous donne le nombre de souches détectées (considérées comme positives quand I≥0,6) en fonction de la concentration en substrat.

**Tableau 2**

| **Substrat** | **Durée d'incubation** | **100 mg/l** | **200 mg/l** | **300 mg/l** |
|---|---|---|---|---|
| Magenta-α-Glu | 24h | 2/8 | 7/8 | 7/8 |
| | 48h | 8/8 | 8/8 | 8/8 |
| RedA-α-Glu | 24h | 1/8 | 5/8 | 6/8 |
| | 48h | 7/8 | 8/8 | 8/8 |
| Rose-α-Glu | 24h | 1/8 | 3/8 | 6/8 |
| | 48h | 7/8 | 8/8 | 8/8 |
| X-α-Glu | 24h | 3/8 | 7/8 | 7/8 |
| | 48h | 7/8 | 7/8 | 7/8 |
| GreenA-α-Glu | 24h | 6/8 | 7/8 | 7/8 |
| | 48h | 8/8 | 8/8 | 7/8 |

Ce tableau met en évidence que la concentration de 200 ou 300 mg/l donne une bonne détection.

### Exemple 3 : Utilisation d'un substrat d'α-glucosidase et d'un substrat pour une activité enzymatique différente

On a répété le mode opératoire décrit dans l'exemple 1 avec le GreenA-α-Glu, à ceci près qu'on a utilisé 133 mg/l de ce substrat, qu'on a ajouté au milieu, après autoclavage, 0,047 g/l d'Aztréonam (ICN), 0,004 g/l d'Amphotéricine B (Squibb) et les composants suivants :
Milieu 1: milieu témoin correspondant au milieu de l'exemple 1 avec le GreenA-α-Glu modifié comme indiqué ci-dessus
Milieu 2: 400 mg/l de 6-chloro-3-indolyl-β-D-cellobioside (Rose-β-cellobioside)
Milieu 3: 400 mg/l de 6-chloro-3-indolyl-β-N-acétylglucosaminide (Rose-β-NAGlu) et 0,5 g/l de N-acétylglucosaminide.

Les résultats sont donnés dans le tableau 3 ci-après, dans lequel C, Co et T sont tels que définis précédemment, avec, dans le cas de la colonne Co, V=vert, G=gris, Mv=mauve, Mg=magenta, B=bleu et Vi=violet, R=rose.

Les souches sont toutes issues de la collection de la Demanderesse.

**Tableau 3**

| | | **Milieu 1** | | **Milieu 2** | | **Milieu 3** | |
|---|---|---|---|---|---|---|---|
| **Souches (n°d'accès)** | **T** | **C** | **Co** | **C** | **Co** | **C** | **Co** |
| *Streptococcus agalactiae* (0101060) | 18 | 0,2 | V | 0,2 | V | 0,3 | V |
| | 24 | 0,2 | V | 0,2 | V | 0,4 | V |
| | 48 | 0,2 | V | 0,2 | V | 0,4 | V |
| *Streptococcus anginosus* (8507046) | 18 | 0,1 | | 0,1 | | 0,1 | |
| | 24 | 0,1 | | 0,1 | | 0,2 | |
| | 48 | 0,2 | | 0,2 | | 0,3 | |
| *Streptococcus pyogenes* (9805062) | 18 | | | | | | |
| | 24 | | | | | | |
| | 48 | | | | | | |
| *Enterobacter cloacae* (0010003) | 18 | 2 | | 2 | R | | |
| | 24 | 2 | G | 2 | R | | |
| | 48 | 3 | V | 3 | R | | |
| *Enterococcus faecium* (0002043) | 18 | 0,2 | | 0,2 | R | 0,4 | R |
| | 24 | 0,3 | | 0,2 | R | 0,5 | R |
| | 48 | 0,3 | | 0,3 | R | 0,5 | R |
| *Enterococcus faecalis* (9001117) | 18 | 1 | V | 0,8 | Vi B | 0,7 | Vi |
| | 24 | 1,3 | V | 1,3 | Vi B | 0,7 | Vi |
| | 48 | 1,7 | V | 1,7 | B | 0,7 | Vi |
| *Listeria monocytogenes* (8309007) | 18 | 0,1 | | 0,1 | | | |
| | 24 | 0,2 | V | 0,2 | V G | 0,1 | |
| | 48 | 0,3 | V | 0,2 | G Vi | 0,4 | |
| *Candida albicans* (9306081) | 18 | | | | | | |
| | 24 | | | | | | |
| | 48 | | | | | | |

Ce tableau met en évidence que l'utilisation d'un deuxième substrat enzymatique permet d'améliorer la spécificité de la détection sans toutefois dégrader la sensibilité de détection des Streptocoques B, et qu'il est possible de distinguer les Streptocoques B des espèces les plus proches et les plus fréquemment rencontrées de manière associée après 24 h d'incubation.

### Exemple 4: Comparaison de la sensibilité de détection de S. agalactiae en utilisant un milieu contenant un substrat d'α-glucosidase selon l'invention et les milieux disponibles dans le commerce

Pour cette étude de sensibilité, on a utilisé un milieu selon l'invention préparé comme décrit dans les exemples précédents mais contenant 0,130 g/l de GreenA-α-Glu, 0,250 g/l de Rose β-D cellobioside, 0,064 g/l d'Aztréonam et 0,004 g/l d'Amphotéricine B (Milieu α-Glu).

Comme milieu de comparaison, on a utilisé le milieu Granada (ref 10 077, BIOLYS, France) (Milieu Granada).

On a ensemencé 69 souches de microorganismes, dont 14 de *Streptococcus agalactiae*, on a laissé incuber à 37°C jusqu'à 24h et à température ambiante au-delà. On a visualisé les colonies comme décrit précédemment. La confirmation des colonies suspectes caractéristiques de Streptocoque B, c'est-à-dire apparaissant vertes a été réalisée par test d'agglutination en utilisant le réactif Slidex Strepto Kit selon les recommandations du fournisseurs (bioMérieux, France). Les colonies non caractéristiques, c'est-à-dire autres que vertes ou ayant la coloration caractéristique mais donnant une réponse négative au test d'agglutination (souches faux positif) ont été identifiées par la Galerie ID 32 Strep (bioMérieux, France).

Les résultats sont exprimés en % de bon diagnostic par rapport à tous les tests en termes de sensibilité et spécificité et sont donnés dans le tableau 4 ci-après, le % de sensibilité correspondant au nombre de vrais positifs détectés sur le milieu par le nombre total de vrais positifs à détecter (*100) et le % de spécificité correspondant au nombre de vrais négatifs détectés sur le milieu par le nombre total de vrais négatifs à détecter (*100).

**Tableau 4**

| | **Sensibilité et spécificité de détection** **de *S. agalactiae* en %** | | | | | |
|---|---|---|---|---|---|---|
| | **Milieu Granada** | | | **Milieu alpha-Glu** | | |
| | **18h** | **24h** | **> 40h** | **18h** | **24h** | **> 40h** |
| **Sensibilité** | 50 | 50 | 50 | 86 | 79 | 93 |
| **Spécificité sans enrichissement** | 100 | 100 | 100 | 100 | 95 | 95 |
| **Spécificité avec enrichissement** | 100 | 100 | 100 | 100 | 100 | 98 |

Les résultats indiqués dans ce tableau mettent en évidence l'amélioration de la sensibilité de détection des Streptocoques B en utilisant le procédé de l'invention. Par ailleurs, ils montrent également que le milieu de détection de l'invention possède également une bonne spécificité, spécificité améliorée après enrichissement du fait d'un passage en bouillon de Todd-Hewitt 18-24 heures à 35-37°C avec ou sans 5% de CO₂ avant ensemencement de la gélose (voir recommandations du CDC (Center for Disease Control), MMWR (Morbidity and Mortality Weekly Report), 16 août 2002, Vol. 51, n° RR-11).

### Exemple 5 : Utilisation du milieu à partir d'échantillons cliniques

Pour cette étude, on a utilisé le milieu selon l'invention tel que préparé comme décrit précédemment dans l'exemple 4.

Un total de 134 échantillons/écouvillons provenant de prélèvements vaginaux ou endocervicaux de femmes enceintes ont été utilisés dans cette étude.

Chaque écouvillon a été émulsifié dans 1 ml d'eau physiologique stérile et 100 µl de cette solution ont été déposés d'une part sur une Gélose Columbia contenant 5% de sang de cheval et d'autre part sur le milieu utilisant le procédé de l'invention. Par ailleurs, 100 µl de la solution précédente ont été utilisés pour inoculer un bouillon Todd Hewitt. Après 20 heures d'incubation à 37°C et en aérobiose, la gélose au sang et le milieu de l'invention ont été ensemencés à partir du bouillon Todd Hewitt puis incubés 20 h à 37°C en aérobiose.

La confirmation des colonies suspectes caractéristiques de Streptocoque B, c'est-à-dire apparaissant vertes, a été réalisée par test d'agglutination en utilisant le réactif Slidex Strepto Kit selon les recommandations du fournisseurs (bioMérieux, France).

Parmi les 134 échantillons, 112 ont été ensemencés sur les milieux gélosés d'une part directement à partir de la suspension en eau physiologique et d'autre part après enrichissement en bouillon Todd Hewitt. Les 22 échantillons restants ont été ensemencés sur les milieux gélosés uniquement directement à partir de la suspension en eau physiologique.

Les résultats, calculés en pourcentage moyen de sensibilité et spécificité, sont présentés dans le tableau 5 ci-après.

**Tableau 5**

| | Gélose Columbia | Gélose invention |
|---|---|---|
| Sensibilité | 95 | 90 |
| Spécificité | 90 | 96 |

Les résultats du tableau montrent que le milieu, utilisé avec des échantillons cliniques, possède une bonne sensibilité (18/20 *Streptococcus agalactiae* détectées) et que la spécificité de détection est améliorée par rapport à un milieu standard (10 résultats faux + contre 24 sur le milieu Columbia au sang), ces résultats étant sensiblement équivalents à ceux obtenus à partir des souches de laboratoire.

## Revendications

1. Procédé de détection spécifique et d'identification de *Streptococcus agalactiae*, **caractérisé en ce qu'**il utilise un milieu réactionnel comprenant au moins un substrat enzymatique d'α-glucosidase.

2. Procédé selon la revendication 1, **caractérisé en ce que** le substrat enzymatique est un substrat à base de dérivés d'indoxyle.

3. Procédé selon la revendication 2, **caractérisé en ce que** la concentration du substrat enzymatique dans le milieu réactionnel est comprise entre 10 et 2000 mg/l.

4. Procédé selon l'une quelconque des revendications 1 à 3, **caractérisé en ce que** le dit milieu réactionnel comprend également un mélange d'aztréonam et d'amphotéricine B.

5. Procédé selon l'une quelconque des revendications 1 à 4, **caractérisé en ce que** le milieu réactionnel comprend également au moins un autre substrat d'activité enzymatique différente, de préférence choisi parmi les substrats de β-cellobiosidase et les substrats de β-glucosaminidase.

6. Procédé de détection spécifique et d'identification de bactérie(s) de l'espèce *Streptococcus agalactiae* dans un échantillon susceptible de contenir cette bactérie, **caractérisé en ce qu'**il comprend les étapes consistant à :
a) inoculer un milieu réactionnel tel que défini dans l'une quelconque des revendications 1 à 5, avec tout ou partie de l'échantillon,
b) incuber le milieu inoculé,
c) révéler la présence d'au moins une activité α-glucosidase seule ou en combinaison avec au moins une autre activité enzymatique différente d'une activité α-glucosidase.

## Claims

1. A method for specifically detecting and identifying *Streptococcus agalactiae,* **characterized in that** a reaction medium comprising at least one α-glucosidase enzymatic substrate is used.

2. The method as claimed in claim 1, **characterized in that** the enzymatic substrate is an indoxyl-derivative-based substrate.

3. The method as claimed in claim 2, **characterized in that** the concentration of the enzymatic substrate in the reaction medium is between 10 and 2000 mg/l.

4. The method as claimed in any one of claims 1 to 3, **characterized in that** said reaction medium also comprises a mixture of aztreonam and amphotericin B.

5. The method as claimed in any one of claims 1 to 4, **characterized in that** the reaction medium also comprises at least one other substrate for different enzymatic activity, preferably chosen from β-cellobiosidase substrates and β-glucosaminidase substrates.

6. A method for specifically detecting and identifying a bacterium or bacteria of the *Streptococcus agalactiae* species in a sample liable to contain this bacterium, **characterized in that** it comprises the steps consisting in:
a) inoculating a reaction medium as defined in any one of claims 1 to 5, with all or part of the sample,
b) incubating the inoculated medium,
c) revealing the presence of at least one α-glucosidase activity alone or in combination with at least one other enzymatic activity other than an α-glucosidase activity.

## Patentansprüche

1. Verfahren zum spezifischen Nachweis und zur Identifikation von *Streptococcus agalactiae*, **dadurch gekennzeichnet, dass** es ein Reaktionsmedium verwendet, das mindestens ein α-Glucosidase-Enzymsubstrat umfasst.

2. Verfahren nach Anspruch 1, **dadurch gekennzeichnet, dass** das Enzymsubstrat ein Substrat auf Basis von Indoxylderivaten ist.

3. Verfahren nach Anspruch 2, **dadurch gekennzeichnet, dass** die Konzentration des Enzymsubstrats im Reaktionsmedium zwischen 10 und 2000 mg/l beträgt.

4. Verfahren nach einem der Ansprüche 1 bis 3, **dadurch gekennzeichnet, dass** das Reaktionsmedium ferner ein Gemisch von Aztreonam und Amphotericin B umfasst.

5. Verfahren nach einem der Ansprüche 1 bis 4, **dadurch gekennzeichnet, dass** das Reaktionsmedium außerdem mindestens ein anderes Substrat für eine unterschiedliche Enzymaktivität umfasst, das vorzugsweise aus den Substraten der β-Cellobiosidase und den Substraten der β-Glucosaminidase ausgewählt ist.

6. Verfahren zum spezifischen Nachweis und zur Identifikation eines Bakteriums (von Bakterien) der Spezies *Streptococcus agalactiae* in einer Probe, die dieses Bakterium enthalten kann, **dadurch gekennzeichnet, dass** es folgende Schritte umfasst:
a) Beimpfen eines Reaktionsmediums, wie in einem der Ansprüche 1 bis 5 definiert, mit der gesamten oder einem Teil der Probe,
b) Inkubieren des beimpften Mediums,
c) Aufdecken des Vorliegens mindestens einer α-Glucosidase-Aktivität allein oder in Kombination mit mindestens einer anderen Enzymaktivität, die von einer α-Glucosidase-Aktivität verschieden ist.
